# EUROPÄISCHE PATENTANMELDUNG

(11) **EP 0 728 485 A2**
(43) Veröffentlichungstag der Anmeldung: **28.08.1996**
(21) Anmeldenummer: 96250044.3
(22) Anmeldetag: 20.02.1996
(51) Int. Cl.: A61K 33/40

(54) **Mittel zur äusserlichen Behandlung von Hufpilz bei Unpaarhufern, insbes.bei Pferden**

(30) Priorität: 23.02.1995 DE 19506347
(71) Anmelder: Sell, Hartmut, 02708 Löbau (DE)
(72) Erfinder: Sell, Hartmut, 02708 Löbau (DE)
(74) Vertreter: Jungblut, Bernhard Jakob, Dr.

(57) **Zusammenfassung**

Die Erfindung lehrt als Mittel zur äußerlichen Behandlung von Hufpilz von Unpaarhufern, insbes. bei Pferden, sowie als Hufpflegemittel eine Emulsion aus Huföl und einer wäßrigen Wasserstoffperoxyd-Lösung mit zumindest einem oxydationsbeständigen Emulgator.

## Beschreibung

Die Erfindung betrifft ein Mittel zur äußerlichen Behandlung von Hufpilz bei Unpaarhufern, insbes. bei Pferden, sowie ein Hufpflegemittel. - Mit äußerlicher Behandlung ist die Applikation auf dem Huf bezeichnet. Bei Hufpilz handelt es sich grundsätzlich um eine Dermatomykose aufgrund einer Infektion mit tierpathogenen Dermatophyten, wobei im wesentlichen der Hufbereich befallen wird. Neueste Erkenntnisse zeigen, daß es sich im einzelnen um eine Erkrankung durch Befall von zwei verschiedenen Arten von opportunistischen Mikroorganismen, die in einer symbiotischen Beziehung zusammen existieren, handelt. Diese Kolonie aus Mikroorganismen umfaßt neben der Dematophytenspezies regelmäßig auch eine Bakteriumspezies. Gemeinsam können sie Enzyme und Exotoxine produzieren, die das Protein und das Kollagen der Hufwand zersetzen. Die von Pferd zu Pferd oft verschiedenen Krankheitsverläufe beruhen darauf, daß die Kolonie sich aus verschieden aggressiven Spezies zusammensetzen kann. Das Behandlungsziel bei der Bekämpfung von Hufpilz ist die Beseitigung insbesondere der parasitischen Dermatophyten aus dem befallenen Gewebe und die Abheilung der Krankheitserscheinungen. Beides zu erreichen ist nicht leicht, da viele fungizide Mittel einerseits durch Eiweiß und andere körpereigene Stoffe deaktiviert werden können und andererseits nicht nur die Dermatophyten, sondern auch den Wirt, d. h. das Pferd, schädigen können. Fungistatische Mittel hemmen die Dermatophyten nur, töten sie aber nicht ab, so daß die Krankheitserscheinungen vorübergehend abklingen können, während die Infektion latent weiter besteht. Die Krankheitserscheinungen bestehen im wesentlichen in einer Versprödung des Hufes, wobei poröse und wenig tragfähige Hufsubstanz gebildet wird. Im fortgeschrittenen Krankheitsverlauf kommt es zu einer fasrigen bis splitterigen Verbreiterung der Hufbasis, zudem ist die Haltekraft der Hufsubstanz für Hufnägel erheblich reduziert.

In dem humanmedizinischen Bereich ist es aus der Praxis bekannt, Dermatomykosen durch äußere Applikation von Präparaten, welche beispielsweise Farbstoffe, Chinolinderivate, Thione, Benzimidazole, Chlotrimazol oder Miconazolnitrat enthalten, zu therapieren. Diese Präparate jedoch sind (ebenso wie andere spezifische Präparate) teuer und stehen deshalb einer breiten Anwendung im veterinärmedizinischen Bereich entgegen. Zudem haben Versuche gezeigt, daß sich der Heilungserfolg mit spezifischen Mitteln oft nicht einstellt aufgrund der verschiedenartigen Zusammensetzungen der Kolonien aus Dematophyten und Bakterien. Für einen wirksamen Einsatz spezifischer Mittel wäre nämlich zunächst eine detailierte pathologische Analyse für das betroffene Pferd erforderlich, gefolgt von einer gezielten Auswahl des spezifischen Mittels. Es versteht sich, daß solche Maßnahmen sehr aufwendig und teuer wären. Im veterinärmedizinischen Bereich ist es aus der Praxis bekannt, eine wäßrige Kaliumpermanganat-Lösung äußerlich auf den befallenen Huf zu applizieren. Eine Therapie mit wäßriger Kaliumpermanganat-Lösung hat sich jedoch als nicht ausreichend erfolgreich gezeigt; zudem treten bei ungewolltem Hautkontakt bei Pferd und/oder behandelndem Mensch störende Verfärbungen, gelegentlich auch Hautreizungen auf. Im übrigen ist anschließend an die Applikation des Mittels eine Abtrocknung des Hufes unter praktisch schmutzfreien Bedingungen und hieran anschließend eine Behandlung mit Huföl erforderlich. Dies ist insgesamt aufwendig.

Der Erfindung liegt die Aufgabe zugrunde, ein Mittel zur Behandlung von Hufpilz bei Unpaarhufern anzugeben, welches gleichzeitig hochwirksam, preiswert in der Herstellung, einfach anwendbar und pflegend ist.

Zur Lösung dieser Aufgabe lehrt die Erfindung eine Emulsion aus Huföl und einer wäßrigen Wasserstoffperoxyd-Lösung mit zumindest einem oxydationsbeständigen Emulgator, vorzugsweise als Mittel zur äußerlichen Behandlung von Hufpilz bei Unpaarhufern, insbes. bei Pferden, oder als Hufpflegemittel. Selbständiger Gegenstand der Erfindung ist die Verwendung einer wäßrigen, Wasserstoffperoxyd-Lösung zur Herstellung eines Mittels für die Behandlung von Hufpilz bei Unpaarhufern, insbes. bei Pferden, sowie die Verwendung einer wäßrigen Wasserstoffperoxyd-Lösung zur Herstellung eines Hufpflegemittels. - Die Erfindung beruht auf der überraschenden Erkenntnis, daß scheinbar die tierpathogenen Dermatophyten ebenso wie ggf. vorliegende Bakterien, welche die Hufpilz-Erkrankungen hervorrufen, mit lediglich einer wäßrigen Wasserstoffperoxyd-Lösung praktisch vollständig abgetötet werden können; und zwar unabhängig von der Zusammensetzung der Kolonie im einzelnen. Dies ist um so überraschender, als es beispielsweise aus der Literaturstelle "Ullmanns Enzyklopädie der technischen Chemie", 4. Auflage, Band 10, 1975, Seite 45, bekannt ist, daß Wasserstoffperoxyd im Rahmen der Humanmedizin noch nicht einmal hinsichtlich seiner bakteriziden Wirkung den Anforderungen genügt. Grundsätzlich lassen sich nämlich die meisten Bakterien erheblich leichter abtöten als Dermatophyten. Mit der Wasserstoffperoxid-Lösung und insbesondere mit der erfindungsgemäßen Emulsion aus Huföl und einer wäßrigen Wasserstoffperoxyd-Lösung werden zudem in einem einzigen Behandlungsschritt nicht nur die Krankheitserreger deaktiviert, sondern auch die hufpilztypischen Krankheitssymptome gelindert bzw. die Hufverbreiterung zurückgebildet. Insofern ist die Erfindung auch als Hufpflegemittel eingesetzt. Huföl im Sinne der Erfindung kann flüssige, aber auch salbenartige Konsistenz aufweisen. Huföl enthält als eine wesentliche Wirkstoffkomponente Lorbeeröl. Häufig beigemischt sind Teerextrakte und, je nach gewünschter Konsistenz, Vaseline sowie Lanolin. In einer bevorzugten Ausführungsform der Erfindung ist die wäßrige Phase der Emulsion eine 2 bis 4 %ige, vorzugsweise 3 %ige Wasserstoffperoxyd-Lösung. Es kann aber auch mit 10 %igem, sogar mit bis zu 70 %igem Wasserstoffperoxidgehalt der wäßrigen Phase gearbeitet werden. Bei weniger als 1 %igem Wasserstoffperoxidgehalt der wäßrigen Phase ist mit nur noch geringem Erfolg zu rechnen.

In einer Ausführungsform der erfindungsgemäßen Ausführungsform ist das Huföl in der wäßrigen Wasserstoffperoxyd-Lösung emulgiert, wobei der Anteil Huföl in der Emulsion weniger als 40 Masse-%, vorzugsweise ca. 10 Masse-% beträgt und wobei die Emulsion flüssige Konsistenz aufweist. In dieser Ausführungsform kann die Emulsion auf den Huf aufgesprüht bzw. aufgetragen werden und dringt ohne weiteres tief in das poröse Hufgewebe ein. In einer anderen Ausführungsform der erfindungsgemäßen Emulsion ist die wäßrige Wasserstoffperoxyd-Lösung in dem Huföl emulgiert, wobei der Anteil Huföl in der Emulsion mehr als 70 Masse-%, vorzugsweise ca. 80 Masse-%, beträgt und wobei die Emulsion dickflüssige bis pastöse Konsistenz aufweist. Eine Emulsion gemäß diesem Ausführungsbeispiel wird ggf. wie eine Salbe auf den Huf aufgetragen und eingerieben.

Als Emulgatoren können verschiedenste übliche Substanzen eingesetzt werden, sofern sie ausreichend oxydationsbeständig gegen Wasserstoffperoxyd und ansonsten tierphysiologisch unbedenklich sind. Hierzu lehrt eine Ausführungsform der Erfindung als Emulgator feinstgemahlene Aktivkohle zu verwenden. Hierbei ist besonders vorteilhaft, daß die Aktivkohle selbst zusätzlich eine beträchtliche bakterizide Wirkung hat. Als Emulgator kann aber auch ein Stoff der Gruppe "Naturstoffemulgatoren" oder Mischungen daraus, vorzugsweise Lanolin und/oder Saponine, verwendet werden. Sofern das Huföl von sich aus ausreichende Mengen an Lanolin mitbringt, braucht kein zusätzlicher Emulgator zugesetzt werden. In Fällen, wo die emulgierende Wirkung der Stoffe aus der Gruppe "Naturstoffemulgatoren" nicht ausreicht, kann stattdessen oder zusätzlich als Emulgator ein Stoff der Gruppe "Nichtionische Emulgatoren" oder Mischungen daraus verwendet werden. Auch andere übliche Emulgatoren, sofern tierphysiologisch unbedenklich, sind einsetzbar. Die Art und die Menge des benötigten Emulgators hängt im wesentlichen von der Beschaffenheit des Huföls sowie der Mengenverhältnisse Huföl zu wäßriger Wasserstoffperoxyd-Lösung ab und kann durch einfache Versuche ermittelt werden. Ist eine pastöse Konsistenz der erfindungsgemäßen Emulsion gewünscht, so kann erforderlichenfalls zusätzlich mit einem Emulsionsverdicker, vorzugsweise Johannisbrotkernmehl, gearbeitet werden. Durch Zusatz von Resorcin in pharmazeutisch üblichen Mengen erreicht man in dem befallenen Gewebe eine zusätzliche Desinfektion von Bakterien.

Je nach verwendetem Emulgator bzw. Huföl kann ein Zerfall des Wasserstoffperoxyds in der wäßrigen Phase der Emulsion stattfinden. Bei längerer Lagerung der Emulsion nimmt dann die fungizide Wirkung des Mittels in störender Weise ab. Für diesen Fall lehrt die Erfindung ein Mittel nach einem der Patentansprüche 1 bis 9 in der Ausführungsform als unmittelbar vor der Behandlung zu mischendes Zweikomponentenpräparat, wobei die beiden Komponenten die Phasen der Emulsion, ggf. mit Zusatzstoffen, sind. Die beiden Komponenten des Mittels werden unmittelbar vor der Behandlung zusammengeführt und durch Rühren, Schütteln o. dgl. miteinander zur Emulsion gemischt. Insbesondere in diesem Fall ist es vorteilhaft, die Emulgatoren so auszuwählen, daß praktisch eine Spontanemulgierung ohne wesentliches mechanisches Rühren stattfindet. Hilfreich hierbei ist die Zugabe von Lösungsvermittlern wie z.B. Alkoholen.

Eine erfindungsgemäße Emulsion läßt sich durch geeignete Auswahl der Emulgatoren und Spontanemulgierung und/oder auf mechanischem Wege herstellen. Als Hilfsmittel zur mechanischen Emulgierung können eingesetzt werden: schnellaufende Rührwerke, Schüttel- oder Schlagmaschinen, Emulgierzentrifugen, Kolloidmühlen, Mischpumpen, Vibratoren, Ultraschallgeneratoren und/oder Homogenisatoren.

Der mit der Erfindung erreichbare Erfolg wird im folgenden anhand eines Ausführungsbeispieles näher erläutert. Ein Reitpferd zeigte eine starke Verbreiterung der Hufbasis sowie eine fasrige bis splittrige Versprödung der Hufsubstanz aufgrund von Hufpilzbefall. Hufeisen ließen sich nicht mehr dauerhaft und sicher befestigen. Auf den befallenen Huf wurden ca. 25 - 50 ml einer wäßrigen 3 %igen Wasserstoffperoxyd-Lösung gleichmäßig aufgesprüht (hierbei sind auch Werte im Bereich von 1%-4%, brauchbar und ein Gehalt von 10% und mehr Wasserstoffperoxid sollte auch noch unbedenklich sein). Nach 15 minutiger Einwirkzeit wurde die Wasserstoffperoxyd-Behandlung wiederholt. Nach dem Abtrocknen des Hufs an der Luft wurde der trockene Huf mit Huföl eingeölt. Die vorstehend beschriebene Behandlung wurde wöchentlich wiederholt. Nach 8 - 12 Wochen der Behandlung hatte sich die Verbreiterung der Hufbasis zurückgebildet und die Hufsubstanz ihre gesunde Struktur wiedergewonnen. Sämtliche Symptome des Hufpilzbefalls waren verschwunden. Nach Absetzen der Behandlung konnte kein Wiederaufleben der Dermatomykose beobachtet werden. Die Durchführung des Ausführungsbeispieles mit einer reinen wäßrigen Wasserstoffperoxyd-Lösung zeigt, daß tatsächlich das Wasserstoffperoxyd selbst der fungizide Wirkstoff ist und nicht etwa ein Bestandteil des Huföls und/oder ein Emulgator. Im folgenden werden verschiedene Beispiele für erfindungsgemäße Emulsionen gegeben.

### Beispiel 1

Eine Mischung aus 33 Vol% handelsüblichem flüssigem Huföl, 42 Vol% 3 %iger wäßriger Wasserstoffperoxidlösung und 25 Vol% des Emulgators Triton X 100 (Octylphenolpoly(ethylenglycolether)ₙ) wurde hergestellt und in einem Schüttelgefäß kräftig und ausreichend lang geschüttelt. Hiernach hatte sich neben einem kleinen Rest wäßriger Phase eine milchfarbene cremeartige Emulsion gebildet, die langzeitstabil war.

### Beispiel 2

Eine Mischung aus 33 Vol% handelsüblichem flüssigem Huföl, 42 Vol% 3 %iger wäßriger Wasserstoffperoxidlösung und 25 Vol% des Emulgators Tween 20 (Polyethoxysorbitanlaurat) wurde hergestellt und in einem Schüttelgefäß kräftig und ausreichend lang geschüttelt. Hiernach hatte sich neben einem kleinen Rest wäßriger Phase eine milchfarbene flüssige Emulsion gebildet, die langzeitstabil war.

### Beispiel 3

Eine Mischung aus 40 Vol% handelsüblichem flüssigem Huföl, 50 Vol% 3 %iger wäßriger Wasserstoffperoxidlösung und 10 Vol% einer Lösung mit 10g des Emulgators SDS (Natriumdodecylsulfat) in 100ml wurde hergestellt und in einem Schüttelgefäß kräftig und ausreichend lang geschüttelt. Hiernach hatte sich neben einem kleinen Rest wäßriger Phase eine milchfarbene flüssige Emulsion gebildet, die langzeitstabil war.

In allen Beispielen zeigte eine halbquantitative Bestimmung des Wasserstoffperoxidgehaltes der Emulsion, daß der Wasserstoffperoxidgehalt innerhalb von 24h nicht extrem unter den aufgrund der Verdünnung ohnehin zu erwartenden Effekt gesunken war.

## Patentansprüche

1. Emulsion aus Huföl und einer wäßrigen Wasserstoffperoxyd-Lösung mit zumindest einem oxydationsbeständigen Emulgator, vorzugsweise als Mittel zur äußerlichen Behandlung von Hufpilz von Unpaarhufern, insbes. bei Pferden, oder als Hufpflegemittel.

2. Emulsion nach Anspruch 1, wobei die wäßrige Wasserstoffperoxyd-Lösung 1 bis 10 Vol%, vorzugsweise 2 bis 4 Vol%, höchstvorzugsweise 3 Vol%, Wasserstoffperoxyd enthält.

3. Emulsion nach Anspruch 1 oder 2, wobei das Huföl in der wäßrigen Wasserstoffperoxyd-Lösung emulgiert ist, wobei der Anteil Huföl in der Emulsion weniger als 40 Masse-%, vorzugsweise ca. 10 Masse-%, beträgt und wobei die Emulsion flüssige Konsistenz aufweist.

4. Emulsion nach Anspruch 1 oder 2, wobei die wäßrige Wasserstoffperoxyd-Lösung in dem Huföl emulgiert ist, wobei der Anteil Huföl in der Emulsion mehr als 70 Masse-%, vorzugsweise ca. 80 Masse-% beträgt und wobei die Emulsion pastöse Konsistenz aufweist.

5. Emulsion nach einem der Ansprüche 1 bis 4, wobei als Emulgator feinstgemahlene Aktivkohle oder ein Stoff der Gruppe "Naturstoffemulgatoren" oder Mischungen daraus, vorzugsweise Lanolin und/oder Saponine, oder ein Stoff der Gruppe "Nichtionische Emulgatoren" oder Mischungen daraus verwendet wird.

6. Emulsion nach einem der Ansprüche 1 bis 5 mit einem Emulsionsverdicker, vorzugsweise Johannisbrotkernmehl.

7. Emulsion nach einem der Ansprüche 1 bis 6 mit Resorcin.

8. Verwendung einer wäßrigen Wasserstoffperoxyd-Lösung zur Herstellung eines Mittels für die äußerliche Behandlung von Hufpilz bei Unpaarhufern, insbes. bei Pferden.

9. Verwendung einer wäßrigen, vorzugsweise 1 bis 10 %igen Wasserstoffperoxyd-Lösung zur Herstellung eines Hufpflegemittels.

10. Mittel nach einem der Ansprüche 1 bis 7 in der Ausführungsform als unmittelbar vor der Behandlung zu mischendes Zweikomponentenpräparat, wobei die beiden Komponenten die Phasen der Emulsion, ggf. mit Zusatzstoffen, sind.
